# EUROPÄISCHE PATENTSCHRIFT

(11) **EP 4 125 742 B1**
(45) Veröffentlichungstag und Bekanntmachung des Hinweises auf die Patenterteilung: **01.05.2024**
(21) Anmeldenummer: 21715215.6
(22) Anmeldetag: 24.03.2021
(51) Int. Cl.: A61F 5/01, A61F 5/30

(54) **PELOTTE UMFASSEND EIN DRUCKELEMENT**
PAD COMPRISING A PRESSURE ELEMENT
COUSSINET COMPRENANT UN ÉLÉMENT DE PRESSION

(30) Priorität: 27.03.2020 DE 102020203999
(43) Veröffentlichungstag der Anmeldung: 08.02.2023
(73) Patentinhaber: Bauerfeind AG, 07937 Zeulenroda-Triebes (DE)
(72) Erfinder: BECK, André, 07937 Zeulenroda-Triebes (DE); BAUERFEIND, Hans Bruno, 07937 Zeulenroda-Triebes (DE)
(74) Vertreter: Wohlfahrt, Jan Günther
(86) Internationale Anmeldenummer: PCT/EP2021/057547
(87) Internationale Veröffentlichungsnummer: WO 2021/191263

(56) Entgegenhaltungen:
- EP-A1- 0 598 291
- DE-A1-102012 105 626
- DE-A1-102014 226 841
- DE-U1-202014 009 969
- FR-A1- 3 037 232

## Beschreibung

Die Erfindung betrifft Pelotten, insbesondere für orthopädische Hilfsmittel und Sporthilfsmittel, umfassend ein Pelottendruckelement. Die Erfindung betrifft auch die Verwendung der erfindungsgemäßen Pelotten in Orthesen, Bandagen oder Kompressionskleidung, sowie Orthesen, Bandagen oder Kompressionskleidung, die die erfindungsgemäßen Pelotten aufweisen.

Pelotten sind in verschiedenen Ausführungsformen und für verschiedene prophylaktische und therapeutische Anwendungen bekannt. Verschiedene Pelotten sind beispielsweise aus der DE 27 22 563 C2, der EP 0 598 291 A1, die den Oberbegriff des Anspruchs 1 offenbart, EP 1 688 107 A1 und der EP 0 600 218 A2 bekannt.

Auch sind Pelotten aus zwei Materialien oder Komponenten aus der DE 297 01 001 U1 bekannt. Die EP 0 496 071 A1 beschreibt eine Druckpelotte aus einem weicheren Material, in dem mindestens ein Friktionskern aus einem harten oder inkompressiblen Material angeordnet ist. Dieser Friktionskern soll einer Friktionsmassage, also einer Massage durch Bewegungsreibung von Schmerzpunkten dienen, die durch die Bewegung des Kerns gegenüber dem Weichgewebe des Pelottenträgers erzeugt wird.

Die Pelotten aus dem Stand der Technik können flächig einen Druck auf das anliegende Gewebe ausüben oder anfallenden Druck auf die Fläche verteilen und/oder abschirmen, jedoch ist mit diesen Pelotten die Ausübung eines spezifischen Drucks, beispielsweise eines stärkeren oder schwächeren Drucks auf spezifische Punkte nicht oder nur unzureichend möglich.

Auch erfolgt der Druck bei Pelotten aus dem Stand der Technik senkrecht auf die Haut. Ein seitlicher Druck, der insbesondere zu einer Bewegung der Druckelemente führt und somit einen Massageeffekt über den einfachen Druck hinaus herbeiführt, ist so nicht möglich.

Das der vorliegenden Erfindung zugrundeliegende technische Problem ist daher die Bereitstellung verbesserter Pelotten, insbesondere solcher Pelotten, die neben einem flächigen Druck einen spezifischen punktgenauen Druck ausüben können. Insbesondere ist das der vorliegenden Erfindung zugrundeliegende technische Problem die Bereitstellung einer Pelotte, die zu einem verbesserten Massageeffekt führt, insbesondere zu einem Massageeffekt durch Bewegung des Druckkörpers oder des Muskels.

Das der Erfindung zugrunde liegende technische Problem wird gelöst durch die Bereitstellung einer Pelotte nach Anspruch 1.

Das der Erfindung zugrunde liegende technische Problem wird gelöst durch die Bereitstellung einer Pelotte für orthopädische Hilfsmittel, umfassend einen Pelottengrundkörper aus einem ersten Material und mindestens einem Pelottendruckelement aus einem zweiten Material, wobei das Pelottendruckelement zumindest teilweise in den Pelottengrundkörper eingebettet ist und wobei das erste Material des Pelottengrundkörpers weicher ist als das zweite Material des Pelottendruckelements, wobei das Pelottendruckelement mindestens zwei Reihen von mindestens je drei Erhebungen aufweist, wobei die mindestens zwei Reihen von Erhebungen mindestens in einem Teilbereich nicht parallel verlaufen und wobei die Erhebungen aus einem Grundelement des Pelottendruckelements herausragen.

Es zeigte sich überraschender Weise, dass Erhebungen, beispielsweise Zapfen oder Noppen, die in Reihen angeordnet sind, somit gezielt auf Muskel wirken können, da sie dem Muskelfaserverlauf folgen und dass dies besonders effektiv ist, wenn mindestens zwei Reihen, also mehrere Reihen von Erhebungen vorhanden sind, die den einzelnen Muskelfaserbündeln eines Muskels folgen.

Ein Skelettmuskel besteht aus mehreren Muskelfaserbündeln, die am Sehnenknochenansatz zusammenlaufen und dort mit dem Knochen verbunden sind. Vom Sehnenknochenansatz aus gesehen verlaufen die Muskelfasern meist erst in einem geringen Winkel auseinanderlaufend um sich auszubreiten und danach in etwa parallel weiterzulaufen.

Der Bereich der mindestens zwei Reihen von Erhebungen in der Pelotte, der nicht parallel läuft, dient der Anlage an den Muskelfaserbereich in Nähe des Sehnenknochenansatzes, in dem die Muskelfasern ebenfalls nicht parallel, sondern auseinander laufen. Die Reihen von Erhebungen können jedoch auch in Bereichen parallel zueinander verlaufen, insbesondere wenn die Reihen verlängert werden, sodass die Erhebungen in den parallel verlaufenden Bereichen auf die in etwa parallel verlaufenden Muskelfasern drücken.

Unter einer Pelotte wird im Zusammenhang mit der vorliegenden Erfindung insbesondere eine Druckpelotte verstanden, deren Form insbesondere durch den Pelottengrundkörper gebildet wird. Der Pelottengrundkörper kann jegliche geeignete Form aufweisen. Herkömmliche Pelotten weisen typischerweise ebene Grundflächen auf. Aus einer solchen Grundfläche können dann Vorsprünge herausragen. Eine erfindungsgemäße Pelotte weist insbesondere eine erste Grundfläche oder Grundoberfläche auf, die bei Verwendung der Pelotte dem Benutzer zugewandt ist. Bevorzugt ist eine Pelotte im Wesentlichen flach ausgestaltet. Eine solche Pelotte weist dann bevorzugt eine zweite Grundfläche oder Grundoberfläche auf, die dem Benutzer bei der Verwendung der Pelotte abgewandt ist. Die zapfenförmigen Erhebungen zeigen vom Pelottengrundkörper in Richtung des Benutzers.

Die typische Verwendung einer Pelotte ist dem Fachmann bekannt. Pelotten, auch die erfindungsgemäßen Pelotten, werden insbesondere dazu verwendet, auf bestimmte Körperpartien, beispielsweise im Bereich des Rückens oder im Bereich der Kniescheibe einen Druck auszuüben. Dabei werden die Pelotten positioniert und über weitere Vorrichtungen, insbesondere Bandagen oder Orthesen auf den entsprechenden Körperbereich gedrückt.

Die Erhebungen, insbesondere Zapfen oder Noppen, der mindestens zwei Reihen sind bevorzugt starr ausgebildet. Dies hat den Vorteil, dass die Bewegung der an den Muskel anschließenden Gelenke eine Verkürzung beziehungsweise Dehnung des Muskels hervorruft. Bei einer starren Ausbildung der Erhebungen bewegt sich der Muskel unter den Erhebungen hinweg und massiert sich somit in vorteilhafter Weise selber.

In einer bevorzugten Ausführungsform weist das Pelottendruckelement drei bis sechs Reihen von mindestens je drei Erhebungen auf.

In einer bevorzugten Ausführungsform weist das Pelottendruckelement drei oder vier Reihen von mindestens je vier Erhebungen auf.

In einer bevorzugten Ausführungsform weisen die Reihen mindestens vier Erhebungen auf. Der Fachmann kann die geeignete Anzahl und den Abstand der Erhebungen ohne weiteres für den angestrebten Verwendungszweck selbst bestimmen.

In einer bevorzugten Ausführungsform weisen die Reihen je vier bis zwölf Erhebungen auf.

In einer bevorzugten Ausführungsform weisen die Reihen je vier bis zehn Erhebungen auf.

In einer bevorzugten Ausführungsform weisen die Reihen je fünf bis neun Erhebungen auf.

In einer bevorzugten Ausführungsform verlaufen zwei benachbarte Reihen der mindestens zwei Reihen von mindestens je drei Erhebungen in dem nicht parallel verlaufenden Teilbereich in einem Winkel von mindestens 3°. In einer bevorzugten Ausführungsform verlaufen die mindestens zwei Reihen von mindestens je drei Erhebungen im Pelottengrundkörper in einem Winkeln von mindestens 3°.

Gemäß der Erfindung verlaufen zwei benachbarte Reihen der mindestens zwei Reihen von mindestens je drei Erhebungen in dem nicht parallel verlaufenden Teilbereich in einem Winkel von mindestens 3° und höchstens 25° zueinander.

Der Fachmann kann ohne weiteres den für ihn geeigneten Winkel der benachbarten Reihen bestimmen, da er die einzelnen Muskelfaserverläufe von unterschiedlichen Muskelpartien kennt. In einer bevorzugten Ausführungsform sind die mindestens zwei Reihen von mindestens je drei Erhebungen im Pelottengrundkörper so positioniert, dass sie im angelegten Zustand der Pelotte im Bereich von Muskelsträngen lokalisiert sind und auf diese Druck ausüben können.

Der Fachmann kann bei der Herstellung der Pelotte je nach Verwendungszweck, also je nach Muskel, auf dessen Muskelfaserstränge durch die Erhebungen Druck ausgeübt werden soll, die geeignete Positionierung und Dimensionierung der Pelotte und des Pelottendruckelements sowie der Erhebungen im Pelottengrundkörper bestimmen.

In einer bevorzugten Ausführungsform weist das Pelottendruckelement mindestens ein Loch auf, wobei mindestens zwei Erhebungen auf dem Pelottendruckelement am Rand des mindestens einen Lochs positioniert sind.

In einer bevorzugten Ausführungsform sind die mindestens zwei Erhebungen über den das Loch überspannenden Steg miteinander verbunden.

In einer bevorzugten Ausführungsform sind am Rand des Lochs mindestens zwei Paare von Erhebungen positioniert, die jeweils über einen das Loch überspannenden Steg miteinander verbunden sind, wobei sich die Stege der beiden Paare von Erhebungen kreuzen, bevorzugt etwa in der Mitte des Lochs kreuzen.

Das Loch mit den zwei Paaren von Erhebungen am Rand und den sich kreuzenden Stegen bildet in bevorzugter Ausführungsform ein Speichenkreuz. Das Loch und bevorzugt das Speichenkreuz sind dabei bevorzugt in der Pelotte so positioniert, dass sie im angelegten Zustand der Pelotte auf dem Sehnenknochenansatz liegen. Durch den Speichenkreuzaufbau sind die Erhebungen beweglich gelagert, insbesondere beweglicher als die Erhebungen der mindestens zwei Reihen. Somit können die am Loch positionierten Erhebungen in vorteilhafter Weise direkt am Sehnenknochenansatz, insbesondere an ihrem vom Grundelement entfernt liegenden Enden zueinander bewegen oder voneinander wegbewegen wenn die Pelotte senkrecht auf die Haut des Patienten gedrückt wird. Das Auseinanderbewegen beziehungsweise Zueinanderbewegen der Erhebungen stellt in vorteilhafter Weise eine seitliche Bewegung dar, die durch den senkrechten Druck der Pelotte auf die Haut verursacht wird und die zu einer verbesserten Massage führt. Dabei stellte sich in vorteilhafter Weise heraus, dass diese seitliche Bewegung chaotisch erfolgt, was den Massageeffekt verstärkt.

Die zusätzlich speichengelagerten Erhebungen federn im Vergleich zu den Erhebungen der Reihen wesentlich freier und beweglicher, und somit insbesondere auch chaotischer. Dieser Effekt ist vorteilhaft, da sich die direkt unter der Hautoberfläche liegenden Mechano-Rezeptoren auf die immer wieder etwas veränderte Federbewegung der Erhebungen schwerer einstellen können. Dies führt zu einer verlängerten Wirkung der Massage.

Während ein auseinander Bewegen der Erhebungen zu einer seitwärts gerichteten Zugbewegung führt, führt das zueinander Bewegen zu einer seitwärts gerichteten Druckbewegung. Es zeigte sich überraschender Weise, dass diese Bewegung sich auch dann deutlich auf die Haut überträgt, wenn die zapfenförmigen Elemente in den weicheren Pelottengrundkörper zumindest teilweise eingebettet, beispielsweise eingegossen, sind.

Somit wird in dieser bevorzugten Ausführungsform durch die Erhebungen der mindestens zwei Reihen ein Druckeffekt auf die Muskelfasern eines Muskels verursacht, der durch die Bewegung des Muskels zu einem Massageeffekt führt und gleichzeitig durch die Bewegung der Erhebungen am Loch ein Massageeffekt auf den Sehnenknochenansatz verursacht. Es werden also zwei verschiedene Massagemöglichkeiten auf zwei verschiedene Angriffspunkte kombiniert, wobei bei der ersten Massageeinheit starre Erhebungen auf sich bewegende Muskelfasern wirken und in der zweiten Massageeinheit sich bewegende Erhebungen auf den starren Sehnenknochenansatz wirken.

In einer bevorzugten Ausführungsform befindet sich eine weitere Erhebung auf und/oder neben dem Kreuzungspunkt der beiden Stege.

In einer bevorzugten Ausführungsform sind die am Rand des Lochs positionierten Erhebungen den mindestens zwei Reihen von mindestens je drei Erhebungen zugeordnet.

In einer bevorzugten Ausführungsform ist das Loch im Endbereich der mindestens zwei Reihen von mindestens je drei Erhebungen positioniert.

In einer bevorzugten Ausführungsform ist das Loch im Pelottengrundkörper so positioniert, dass die am Rand des Lochs positionierten Erhebungen im angelegten Zustand der Pelotte im Bereich eines Sehnenknochenansatzes lokalisiert sind und auf diesen Druck ausüben können.

Bevorzugt sind also die Erhebungen der Reihen und das Loch beziehungsweise die Erhebung des Lochs so zueinander in der Pelotte positioniert, dass die Erhebungen der Reihen auf die Muskelfasern wirken und dem Verlauf der Muskelfasern folgen, während das Loch und die dem Loch zugeordneten Erhebungen auf den Sehnenknochenansatz wirken. Wenn die Reihen mit Erhebungen im Bereich des Lochs anfangen, wirken die Erhebungen in den Reihen dementsprechend auf den Muskelfaserbereich, der nahe des Sehnenknochenansatzes liegt, also auf den Endbereich eines Muskels. Selbstverständlich können die Reihen verlängert werden, sodass sie auch auf weiter vom Sehnenknochenansatz entferntere Bereiche der Muskelfasern wirken.

Natürlich kann auch vorgesehen sein, dass mehrere Einheiten von nebeneinander verlaufenden Reihen vorgesehen sind, sodass einzelne Einheiten auf unterschiedliche Muskelfaserverläufe wirken können. Beispielsweise kann vorgesehen sein, dass bei einer Ellenbogenpelotte das Loch mit den zugeordneten Erhebungen im Bereich des Ellenbogens liegt und mindestens zwei Reihen von Erhebungen in Richtung Unterarm verlaufen, also auf Muskelfasern des Unterarms wirken, während mindestens zwei weitere Reihen von Erhebungen in Richtung Oberarm positioniert sind, also auf Muskelfasern des Oberarms wirken. Die verschiedenen Einheiten von Erhebungsreihen können auch beweglich zueinander positioniert sein.

In einer bevorzugten Ausführungsform weist das Pelottendruckelement mindestens zwei zusätzliche Reihen von mindestens je drei Erhebungen auf, wobei die mindestens zwei zusätzlichen Reihen von Erhebungen um etwa 180° versetzt zu den ersten mindestens zwei Reihen von Erhebungen verlaufen.

Bevorzugt sind dabei also die erfindungsgemäßen mindestens zwei Reihen an Erhebungen gespiegelt, besonders bevorzugt im Bereich des bevorzugten Lochs gespiegelt.

In einer bevorzugten Ausführungsform weist das Pelottendruckelement mindestens zwei zusätzliche Reihen von mindestens je drei Erhebungen auf, wobei die mindestens zwei zusätzlichen Reihen von Erhebungen versetzt in einem Winkelbereich von 75° bis 105° und/oder versetzt in einem Winkelbereich von 165° bis 205° zu den ersten mindestens zwei Reihen von Erhebungen verlaufen.

In einer bevorzugten Ausführungsform weist das Pelottendruckelement eine erste Einheit von mindestens zwei Reihen von mindestens je drei Erhebungen, eine zweite Einheit von mindestens zwei Reihen von mindestens je drei Erhebungen und eine dritte Einheit von mindestens zwei Reihen von mindestens je drei Erhebungen auf, wobei die mindestens zwei Reihen der ersten Einheit und die mindestens zwei Reihen der zweiten Einheit in einem Winkelbereich von 75° bis 105° versetzt sind und die mindestens zwei Reihen der zweiten Einheit und die mindestens zwei Reihen der dritten Einheit in einem Winkelbereich von 75° bis 105° versetzt sind. Besonders bevorzugt sind dabei die zwei Reihen der ersten Einheit und die zwei Reihen der dritten Einheit in einem Winkelbereich von 165° bis 205° zueinander versetzt.

Eine solche Pelotte mit zwei oder drei Einheiten von Reihen mit Erhebungen, die in einem bestimmten Winkel zueinander versetzt sind, kann in vorteilhafter Weise beispielsweise als Schulterpelotte eingesetzt werden. Dabei kann eine Einheit von Reihen in Richtung Brust verlaufen, eine Einheit von Reihen in Richtung Rücken und eine Einheit von Reihen in Richtung Arm.

In einer weiteren alternativen Ausführungsform können mehrere Einheiten von Erhebungsreihen, beispielsweise vier Einheiten von je zwei oder drei Reihen von Erhebungen, strahlenförmig voneinander weglaufen. Bevorzugt befindet sich in dem Bereich, in dem die Strahlen zusammenlaufen, das bevorzugte Loch mit überspannenden Stegen und Erhebungen am Rand des Loches und auf den Stegen. Eine solche Ausführungsform der erfindungsgemäßen Pelotte kann beispielsweise im Hüftbereich oder im Gesäßbereich verwendet werden.

Für alle Ausführungsformen der Pelotte gilt, dass die einzelnen Einheiten aus mindestens zwei Reihen von mindestens je drei Erhebungen so aufgebaut sein können, wie die einzelne Grundeinheit an Reihen von Erhebungen, also beispielsweise mindestens zwei Reihen von Erhebungen umfassen können, aber auch drei Reihen, vier Reihen oder mehr Reihen von Erhebungen umfassen können und die Anzahl der Erhebungen pro Reihe mindestens drei betragen kann, aber auch mehr Erhebungen pro Reihe, beispielsweise vier Erhebungen, fünf Erhebungen, sechs Erhebungen, sieben Erhebungen, acht Erhebungen oder mehr Erhebungen vorgesehen sein können.

In einer bevorzugten Ausführungsform ist das Grundelement des Pelottendruckelements stegförmig oder plattenförmig ausgebildet. Das bevorzugte Loch befindet sich dabei dann im Steg oder in der Platte. Die Erhebungen ragen aus dem Steg oder der Platte heraus.

In einer bevorzugten Ausführungsform sind die Erhebungen zapfenförmig oder noppenförmig. In einer bevorzugten Ausführungsform sind die Erhebungen der Reihen zapfenförmig oder noppenförmig. In einer bevorzugten Ausführungsform sind die Erhebungen am Loch zapfenförmig oder noppenförmig.

In einer bevorzugten Ausführungsform ist zumindest ein Teilbereich der Erhebungen in den Pelottengrundkörper eingebettet. Bevorzugt sind die Erhebungen komplett in den Pelottengrundkörper eingebettet. Besonders bevorzugt ist das gesamte Pelottendruckelement, also die Erhebungen und das Grundelement in den Pelottengrundkörper eingebettet, bevorzugt ist also das Pelottendruckelement vom Pelottengrundkörper ganz umschlossen, beispielsweise in den Pelottengrundkörper eingegossen.

Durch die bevorzugte vollständige Ummantelung des mindestens einen Pelottendruckelements mit dem Material des Pelottengrundkörpers ergibt sich nicht nur der beschriebene anwendungstechnische Vorteil, sondern auch in vorteilhafter Weise eine einfachere Herstellungsmöglichkeit der erfindungsgemäßen Pelotte, da somit die zweite Grundfläche des Pelottengrundkörpers, die dem Benutzer abgewandt ist, ohne weiteres plan herstellbar ist

Bevorzugt ist eine Pelotte, umfassend einen Pelottengrundkörper aus einem Material mit einer ersten Härte und mindestens ein erfindungsgemäßes Pelottendruckelement aus einem Material mit einer zweiten Härte, wobei der Pelottengrundkörper eine erste, dem Benutzer zugewandte Grundfläche und eine zweite, dem Benutzer abgewandte Grundfläche aufweist, wobei die Oberfläche des mindestens einen Pelottendruckelements vollständig von dem Material des Pelottengrundkörpers umgeben ist.

Bevorzugt ist das Pelottendruckelement dabei ein Druckverstärkungselement. Ein Druckverstärkungselement dient einem punktuellen stärkeren Druck als der durch den Pelottengrundkörper ausgeübte Flächendruck.

In einer bevorzugten Ausführungsform sind das Grundelement und die Erhebungen einstückig ausgebildet, insbesondere sind sie aus demselben Material gebildet.

In einer bevorzugten Ausführungsform weist der Pelottengrundkörper eine erste, dem Benutzer zugewandte Grundfläche und eine zweite, dem Benutzer abgewandte Grundfläche auf, wobei die Erhebungen von dem im Bereich der zweiten, dem Benutzer abgewandten Grundfläche positionierten Grundelements in Richtung der ersten, dem Benutzer zugewandten Grundfläche ragen.

Die Erhebungen zeigen vom Pelottengrundkörper in Richtung des Benutzers, der Kopf liegt also in Richtung der ersten Grundfläche des Pelottengrundkörpers und der Fuß und das Grundelement in Richtung der zweiten Grundfläche des Pelottengrundkörpers.

In einer bevorzugten Ausführungsform haben die mindestens Erhebungen eine Höhe von mindestens 1 mm und höchsten 10 mm, beispielsweise von etwa 3 mm. In einer bevorzugten Ausführungsform haben die Erhebungen eine Höhe von mindestens 2 mm. In einer bevorzugten Ausführungsform haben die Erhebungen eine Höhe von mindestens 3 mm. Die Höhe bemisst sich von dem Grundelement aus.

Bevorzugt ist die Pelotte zumindest teilweise viskoelastisch, weist also bevorzugt mindestens einen viskoelastischen Teilbereich auf. Insbesondere ist die Pelotte bevorzugt zumindest im Bereich des Pelottendruckelements viskoelastisch.

Bevorzugt weist der Pelottengrundkörper zumindest im Bereich des Pelottendruckelements ein viskoelastisches Material auf. Bevorzugt ist der Pelottengrundkörper zumindest in einem Teilbereich viskoelastisch. Bevorzugt ist der Pelottengrundkörper viskoelastisch. Bevorzugt besteht der Pelottengrundkörper aus einem viskoelastischen Material.

Bevorzugt ist das Material des Pelottengrundkörpers Kunststoff, Silikon oder Gummi. Einem Fachmann sind aber auch weitere geeignete Materialien für einen Pelottengrundkörper aus dem Stand der Technik bekannt.

Beispielsweise kann es sich bei dem Material um thermoplastische Elastomere handeln. Geeignet sind beispielsweise auch Polyuretane.

Bevorzugt ist das Material des Pelottengrundkörpers biegbar und/oder dehnbar, so dass der Pelottengrundkörper beim Anlegen der Pelotte sich an die Körperform des Benutzers anpassen kann.

Bevorzugt ist das Pelottengrundkörpermaterial kein Textilmaterial. Der Pelottengrundkörper kann in einer alternativen Ausführungsform jedoch auch zusätzlich von einer textilen Ummantelung umgeben sein oder anderweitig beschichtet sein.

Das Pelottengrundkörpermaterial kann natürlich auch aus Mischungen von Materialen, insbesondere von mindestens zwei der genannten Materialen bestehen.

Bevorzugt ist das Material des Pelottendruckelements ein biegsames Material, insbesondere ein biegsamer Kunststoff. In einer bevorzugten Ausführungsform ist das Material des mindestens einen Pelottendruckelements Kunststoff, Silikon oder Gummi. Das Material des mindestens einen Pelottendruckelements kann natürlich auch aus Mischungen von Materialen, insbesondere von mindestens zwei der genannten Materialen bestehen.

Somit kann die erfindungsgemäße Zapfen-/Grundelementkonstruktion in vorteilhafter Weise einfach beispielsweise mittels Spritzgusstechnik hergestellt werden.

Erfindungsgemäß ist das erste Material des Pelottengrundkörpers weicher ist als das zweite Material des Pelottendruckelements.

In einer bevorzugten Ausführungsform hat das Material des Pelottengrundkörpers eine Shorehärte von mindestens 10 Shore OO und höchstens 50 Shore OO.

Bevorzugt hat das Material des Pelottengrundkörpers, also das erste Material eine Härte von höchstens 45 Shore OO, bevorzugt höchsten 40 Shore OO, besonders bevorzugt höchsten 30 Shore OO. Bevorzugt hat das Material des Pelottengrundkörpers, also das erste Material eine Härte von höchstens 25 Shore OO, bevorzugt höchsten 20 Shore OO, besonders bevorzugt höchsten 19 Shore OO. Bevorzugt hat das Material des Pelottengrundkörpers eine Härte von mindestens 10 Shore OO, insbesondere mindestens 14 Shore OO, besonders bevorzugt von mindestens 15 Shore OO.

In einer bevorzugten Ausführungsform hat das Material des Pelottendruckelements eine Shorehärte von mindestens 10 Shore A, bevorzugt mindestens 14 Shore A und höchstens 80 Shore A. In einer bevorzugten Ausführungsform hat das Material des Pelottendruckelements hat eine Shorehärte von mindestens 20 Shore A und höchstens 80 Shore A.

In einer bevorzugten Ausführungsform weist das Material des mindestens einen Pelottendruckelements eine Härte von mindestens 20 Shore A und höchstens 60 Shore A auf.

In einer bevorzugten Ausführungsform weist das Material des mindestens einen Pelottendruckelements eine Härte von mindestens 20 Shore A, mehr bevorzugt von mindestens 25 Shore A auf.

In einer bevorzugten Ausführungsform weist das Material des mindestens einen Pelottendruckelements eine Härte von höchstens 50 Shore A, auf. In einer bevorzugten Ausführungsform weist das Material des mindestens einen Pelottendruckelements eine Härte von höchstens 49 Shore A, mehr bevorzugt von höchstens 45 Shore A auf.

Bevorzugt weist das Material des mindestens einen Pelottendruckelements eine Härte von mindestens 35 bis höchsten 45 Shore A auf. Bevorzugt weist das Material des mindestens einen Pelottendruckelements eine Härte von etwa 40 Shore A auf.

In einer bevorzugten Ausführungsform hat das Material des Pelottengrundkörpers eine Shorehärte von mindestens 10 Shore OO und höchstens 50 Shore OO und/oder das Material des Pelottendruckelements hat eine Shorehärte von mindestens 10 Shore A, bevorzugt mindestens 14 Shore A und höchstens 80 Shore A.

In einer bevorzugten Ausführungsform ist die Pelotte eine Ellenbogenpelotte, eine Schulterpelotte, eine Kniepelotte, eine Sprunggelenkspelotte, eine Rückenpelotte, eine Pelotte für den Armbereich oder eine Pelotte für den Bauchbereich.

In einer bevorzugten Ausführungsform ist die Pelotte eine Ellenbogenpelotte oder eine Schulterpelotte. In einer bevorzugten Ausführungsform ist die Pelotte eine Extremitätenpelotte, insbesondere Armpelotte. In einer bevorzugten Ausführungsform ist die Pelotte eine Ellenbogenpelotte, eine Schulterpelotte, eine Kniepelotte, eine Sprunggelenkspelotte oder eine Pelotte für den Armbereich. In einer alternativen Ausführungsform ist die Pelotte eine Rückenpelotte oder eine Pelotte für den Bauchbereich.

In einer alternativen Ausführungsform ist die Pelotte eine Schulterpelotte. In einer weiteren alternativen Ausführungsform ist die Pelotte eine Pelotte für den Hüftbereich oder für den Gesäßbereich.

In einer bevorzugten Ausführungsform ist die Pelotte eine Ellenbogenpelotte, eine Schulterpelotte, eine Hüftpelotte oder eine Pelotte für den Gesäßbereich.

Bevorzugt ist die Pelotte keine Fußpelotte und/oder keine Schuhsohle. Bevorzugt ist die Pelotte keine Schuhsohle. Die erfindungsgemäße Pelotte ist bevorzugt keine Schuheinlage oder Fußeinlage.

In einer bevorzugten Ausführungsform ist die Pelotte keine Rückenpelotte.

In einer bevorzugten Ausführungsform ist die Pelotte eine Ellenbogenpelotte, wobei der Grundkörper der Pelotte ein Loch zum Positionieren der Pelotte aufweist, also ein Grundkörperpositionierungsloch.

Das Loch im Grundkörper eignet sich in vorteilhafter Weise um die Positionierung der Pelotte am Körper des Patienten zu definieren und die Pelotte zu positionieren. Beispielsweise kann das Loch im Grundkörper so positioniert und dimensioniert sein, dass es im Ellenbogenbereich den Knochenansatz umfasst und so die Positionierung der Pelotte fixiert wird.

Die vorliegende Erfindung betrifft auch eine Orthese, Bandage oder Kompressionsbekleidung umfassend eine erfindungsgemäße Pelotte.

Die Pelotte eignet sich in vorteilhafter Weise nicht nur für medizinische Produkte, beispielsweise Orthesen oder Bandagen, sondern auch für andere Kompressionsbekleidung, beispielsweise im Sportbereich. Die Pelotte kann beispielsweise auch bei Sportbekleidung, insbesondere Sportkompressionsbekleidung verwendet werden.

Weitere bevorzugte Gegenstände der Erfindung ergeben sich aus den Unteransprüchen und den Nebenansprüchen.

Die Erfindung wird anhand der nachfolgenden Figuren näher beschrieben, ohne dass die dort dargestellten Ausführungsformen der Erfindung beschränkt zu verstehen sind.
- Figur 1: zeigt eine erfindungsgemäße Pelotte.
- Figur 2: zeigt das Pelottendruckelement der Pelotte aus Figur 1.
- Figur 3: zeigt die Positionierung einer erfindungsgemäßen Ellenbogenpelotte am Arm eines Menschen.
- Figur 4: zeigt eine Ausführungsform der erfindungsgemäßen Pelotte mit drei Einheiten von Erhebungsreihen.
- Figur 5a: zeigt eine weitere Ausführungsform der erfindungsgemäßen Pelotte mit drei Einheiten von Erhebungsreihen.
- Figur 5b: zeigt die Ausführungsform von Figur 5a in der Schrägansicht.
- Figur 6: zeigt eine Ausführungsform der erfindungsgemäßen Pelotte mit zwei Einheiten von Erhebungsreihen.
- Figur 7: zeigt eine weitere Ausführungsform der erfindungsgemäßen Pelotte mit strahlenförmig verlaufenden Erhebungsreihen.
- Figur 8: zeigt eine erfindungsgemäße Pelotte auf einer Bandagengrundmaschenware.

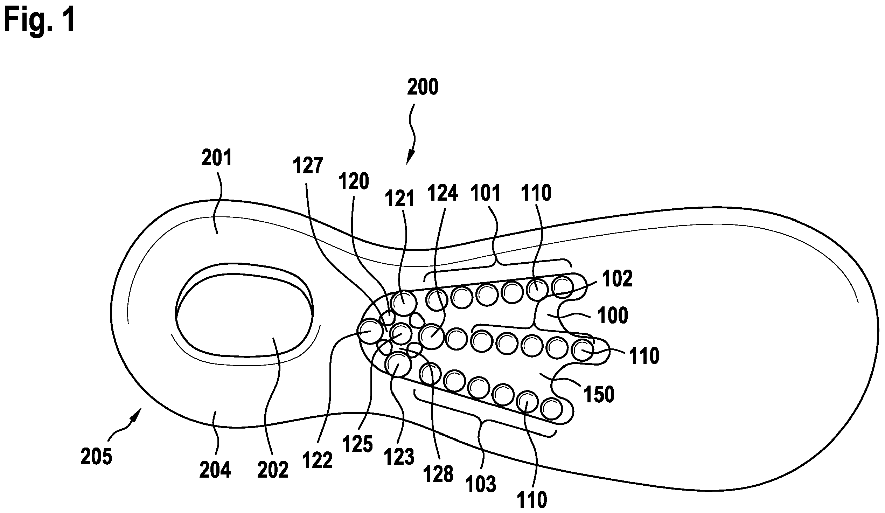

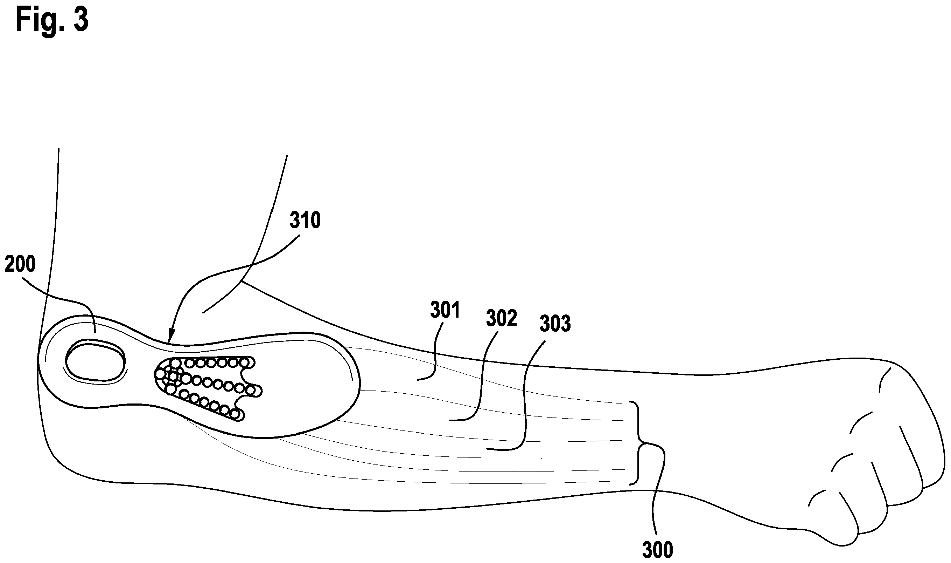

Figur 1 zeigt eine erfindungsgemäße Pelotte (200), im vorliegenden Fall eine Ellenbogenpelotte. Die Pelotte (200) besteht aus einem weicheren Pelottengrundkörper (201) und einem in den Pelottengrundkörper (201) eingebetteten härteren Pelottendruckelement (100). Der Pelottengrundkörper (201) hat einen ringförmigen Bereich, der ein Loch (202) bildet, das in vorteilhafterweise zur Positionierung der Pelotte (200) an einem Ellenbogen genutzt werden kann wie in Figur 3 gezeigt. Die Pelotte (200) hat eine dem Körper des Trägers zugewandte Vorderseite (204) und eine dem Körper des Trägers abgewandte Rückseite (205). Das Pelottendruckelement (100) ist vollständig in einem zungenförmigen Abschnitt des Pelottengrundkörpers (201) eingebettet, und zwar so, dass er noppenförmige Erhebungen (110) von dem Grundelement (150) des Pelottendruckelements (100) in Richtung Vorderseite (204) der Pelotte (200) zeigen, also in Richtung des Arms des Trägers.

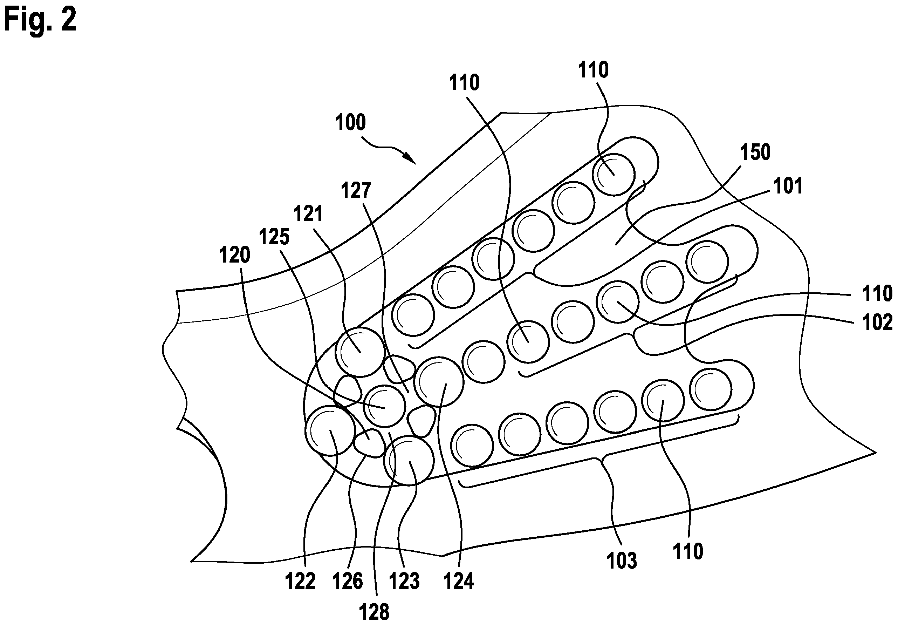

Das Pellotendruckelement (100) der Pelotte (200) aus Figur 1, das in Figur 2 einzeln gezeigt wird, besteht aus einem plattenförmigen Grundelement (150) mit von diesem herausragenden noppenförmigen Erhebungen (110). Auf dem Grundelement (150) sind eine erste Reihe (101), eine zweite Reihe (102) und eine dritte Reihe (103) von Erhebungen (110) gebildet, die zueinander strahlenförmig, also nicht parallel verlaufen. Am Endbereich dieser Reihen (101, 102, 103) befindet sich im Grundelement (150) ein Loch (120). Am Rand (126) des Lochs (120) sind vier weitere Erhebungen (121, 122, 123, 124) positioniert. Das Loch wird von zwei Stegen (127, 128) überspannt in deren Kreuzungspunkt sich eine weitere Erhebung (125) befindet. Durch das Loch (120) sind die beim Loch (120) befindlichen Erhebungen (121, 122, 123, 124, 125) beweglicher als die in den Reihen (101, 102, 103) auf dem Grundelement (150) angeordneten Erhebung (110).

Figur 3 zeigt die erfindungsgemäße Ellenbogenpelotte (200) aus Figur 1 beim Tragen am Arm. Die zungenförmige Pelotte läuft entlang der Unterarmmuskulatur (300). Dabei folgen die drei Reihen von Erhebungen jeweils dem Verlauf verschiedener Muskelfasern (301, 302, 303), sodass die Erhebungen über einen längeren Verlauf der Muskelfasern (301, 302, 303) auf diese einen Druck ausüben können. Das Loch im Pelottendruckelement mit den sich kreuzenden Stegen und den am Loch und auf den Stegen befindlichen Erhebungen befindet sich im Bereich des Sehnenknochenansatzes (310) des Muskels (300), sodass die am Loch befindlichen Erhebungen auf den Sehnenknochenansatz (310) drücken. Durch das Loch und die Stege federn diese Erhebungen im Vergleich zu den Erhebungen der Reihen wesentlich freier und beweglicher. Somit wird durch die Pelotte auf die Muskelfasern (301, 302, 303) durch die dortigen Erhebungen der Pelotte ein anderer, insbesondere stärkerer Druck ausgeübt als durch die beweglicheren Erhebungen im Bereich des Lochs auf den Sehnenknochenansatz (310). Durch die zwei verschiedenen Arten von Druck in den zwei verschiedenen Bereichen kommt es zu einer synergistischen Wirkung der beiden Erhebungsarten. Das Loch des Pelottengrundkörpers kann in vorteilhafter Weise den Kopf des Oberarmknochens umschließen und so eine genaue und sichere Positionierung der Pelotte (200) sicherstellen.

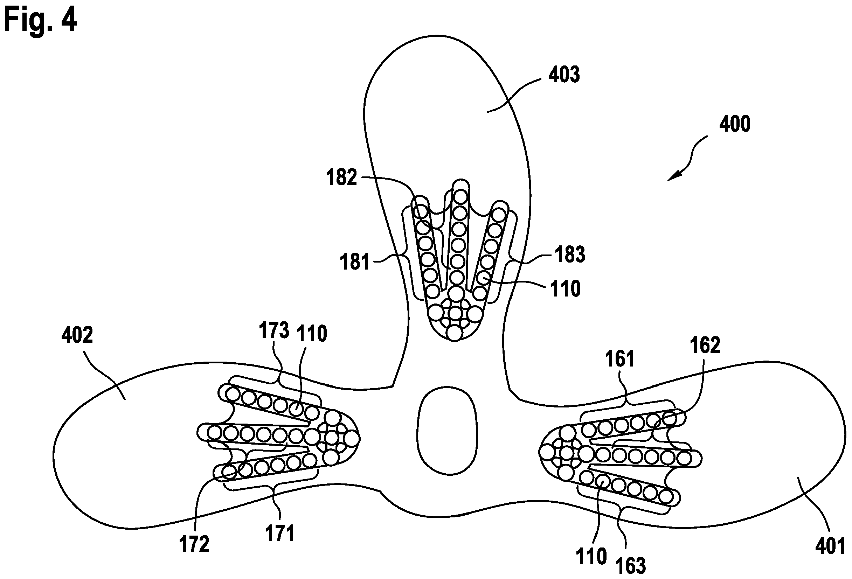

Figur 4 zeigt eine erfindungsgemäße Ellenbogenpelotte (400) zur Verwendung im Schulterbereich. Der Pelottengrundkörper besteht aus drei Zungen (401, 402, 403), die jeweils ein Pelottendruckelement mit jeweils drei Reihen (161, 162, 163, 171, 172, 173, 181, 182, 183) von Erhebungen (110) aufweisen. Die Pelotte (400) kann mit dem Loch im Pelottengrundkörper den oberen Kopf des Oberarmknochens umfassen, sodass sich die Reihen (161, 162, 163) der ersten Zunge (401) im Bereich der Brust befinden, die Reihen (181, 182, 183) der dritten Zunge (403) im Bereich des Oberarms befinden und die Reihen (171, 172, 173) der zweiten Zunge (402) im Bereich des Rückens befinden, so dass in allen drei Bereichen ein Druck auf Muskelfasern erzeugt werden kann.

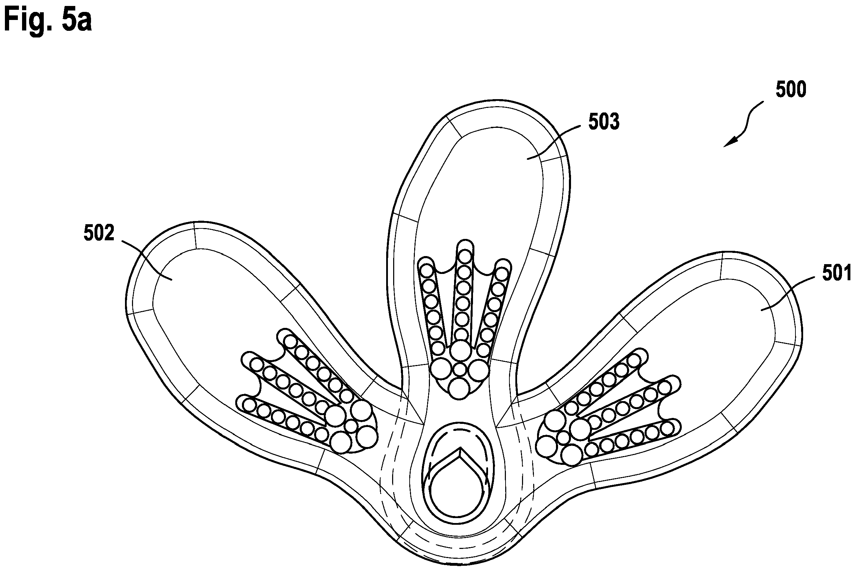

Figur 5a zeigt eine alternative Ausführungsform (500) der Schulterpelotte aus Figur 4, wobei die drei Zungen (501, 502, 503) in einem anderen Winkel zueinander stehen. Die Funktion der Pelotte aus Figur 5a ist die gleiche wie die der Pelotte (400) aus Figur 4.

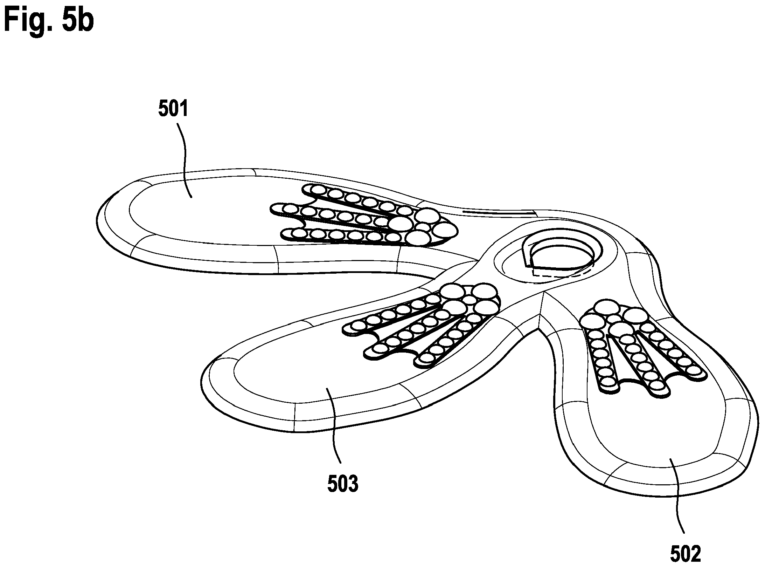

Figur 5b zeigt die Pelotte aus Figur 5a mit den drei Zungen (501, 502, 503) in Schrägansicht.

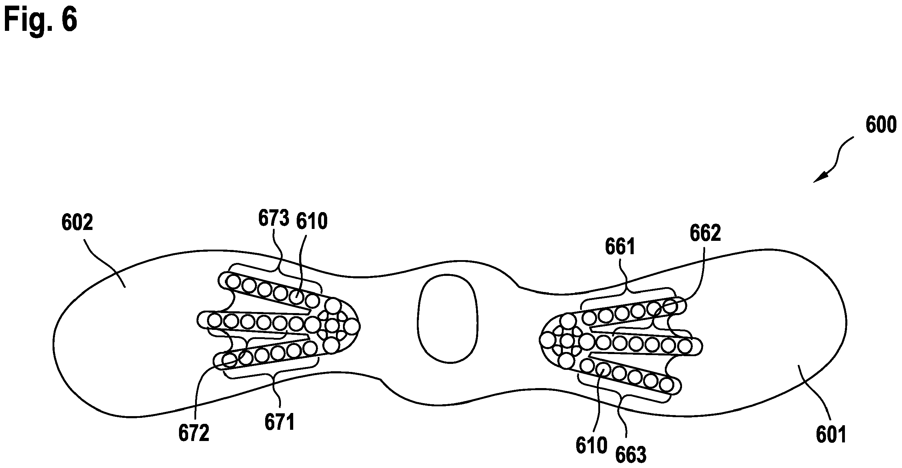

Figur 6 zeigt eine erfindungsgemäße Pelotte (600), beispielsweise eine Armpelotte, mit zwei Zungen (601, 602). Die erste Zunge (601) kann mit ihren drei Reihen (661, 662, 663) von Erhebungen (610) entlang eines Muskels des Unterarms verlaufen, die zweite Zunge (602) kann mit ihren drei Reihen (671, 672, 673) von Erhebungen (610) entlang eines Muskels des Oberarms verlaufen.

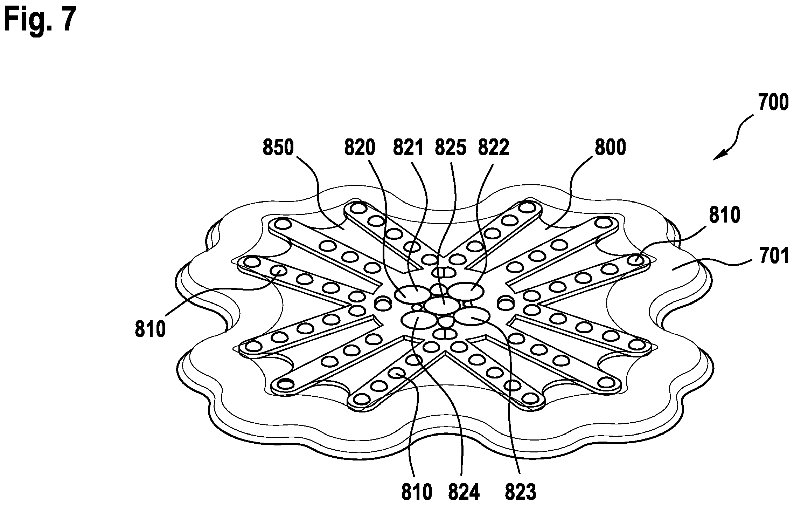

Figur 7 zeigt eine Ausführungsform der erfindungsgemäßen Pelotte (700) für den Hüftbereich oder den Gesäßbereich. Die Pelotte (700) hat einen sternförmigen Pelottengrundkörper (701) in den ein härteres Druckelement (800) mit sternförmigen Grundelement (850) eingebettet ist. Das Druckelement (800) weist insgesamt zwölf Reihen von Erhebungen (810) auf, die von der Mitte der Pelotte (700) strahlenförmig auseinanderlaufen. Dabei sind jeweils drei Reihen als Einheit zusammengefasst. Im Zentrum des Pelottendruckelements (800) befindet sich ein Loch (820) mit zwei Stegen. Am Rand des Lochs (820) befinden sich vier beweglichere Erhebungen (821, 822, 823, 824) sowie eine weitere beweglichere Erhebung in der Mitte des Lochs (825).

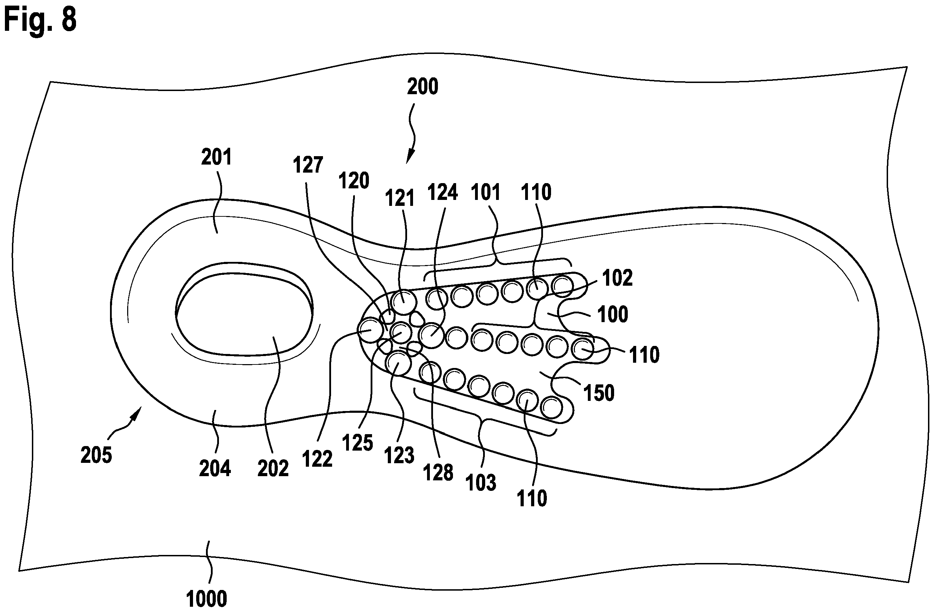

Figur 8 zeigt die erfindungsgemäße Pelotte (200) aus Figur 1, die einer Bandagenmaschenware (1000) zugeordnet ist, beispielsweise einer röhrenförmigen Armbandage. Die Pelotte (200) ist mit der dem Benutzer abgewandten Grundfläche (205) auf der Bandagenmaschenware (1000) befestigt

Die genaue Beschreibung der Pelotte (200) mit den entsprechenden Bezugszeichen kann der Beschreibung zu Figur 1 entnommen werden.

## Patentansprüche

1. Pelotte (200, 400, 600, 700) für orthopädische Hilfsmittel oder Kompressionsbekleidung, umfassend einen Pelottengrundkörper (201, 401, 402, 403, 501, 502, 503, 601, 602, 701) aus einem ersten Material und mindestens einem Pelottendruckelement (100, 800) aus einem zweiten Material, wobei das erste Material des Pelottengrundkörpers (201, 401, 402, 403, 501, 502, 503, 601, 602, 701) weicher ist als das zweite Material des Pelottendruckelements (100, 800), wobei das Pelottendruckelement (100, 800) mindestens zwei Reihen (101, 102, 103, 161, 162, 163, 171, 172,173, 181, 182, 183, 661, 662, 663, 671, 672, 673) von mindestens je drei Erhebungen (110, 610, 810) aufweist, wobei die mindestens zwei Reihen (101, 102, 103, 161, 162, 163, 171, 172,173, 181, 182, 183, 661, 662, 663, 671, 672, 673) von Erhebungen (110, 610, 810) mindestens in einem Teilbereich nicht parallel verlaufen und wobei die Erhebungen (110, 610, 810) aus einem Grundelement (150, 850) des Pelottendruckelements herausragen **dadurch gekennzeichnet, dass** das Pelottendruckelement (100, 800) zumindest teilweise in den Pelottengrundkörper (201, 401, 402, 403, 501, 502, 503, 601, 602, 701) eingebettet ist und dass zwei benachbarte Reihen (101, 102, 103, 161, 162, 163, 171, 172,173, 181, 182, 183, 661, 662, 663, 671, 672, 673) der mindestens zwei Reihen (101, 102, 103, 161, 162, 163, 171, 172,173, 181, 182, 183, 661, 662, 663, 671, 672, 673) von mindestens je drei Erhebungen (110, 610, 810) in dem nicht parallel verlaufenden Teilbereich in einem Winkel von mindestens 3° und höchstens 25° zueinander verlaufen.

2. Pelotte (200, 400, 600, 700) nach Anspruch 1, **dadurch gekennzeichnet, dass** das Pelottendruckelement (100, 800) drei bis sechs Reihen (101, 102, 103, 161, 162, 163, 171, 172,173, 181, 182, 183, 661, 662, 663, 671, 672, 673) von mindestens je drei Erhebungen (110, 610, 810) aufweist.

3. Pelotte (200, 400, 600, 700) nach einem der vorhergehenden Ansprüche, **dadurch gekennzeichnet, dass** die Reihen (101, 102, 103, 161, 162, 163, 171, 172,173, 181, 182, 183, 661, 662, 663, 671, 672, 673) je vier bis 12 Erhebungen (110, 610, 810) aufweisen.

4. Pelotte (200, 400, 600, 700) nach einem der vorhergehenden Ansprüche, **dadurch gekennzeichnet, dass** die mindestens zwei Reihen (101, 102, 103, 161, 162, 163, 171, 172,173, 181, 182, 183, 661, 662, 663, 671, 672, 673) von mindestens je drei Erhebungen (110, 610, 810) im Pelottengrundkörper (201, 401, 402, 403, 501, 502, 503, 601, 602, 701) so positioniert sind, dass sie im angelegten Zustand der Pelotte (200, 400, 600, 700) im Bereich von Muskelsträngen (301, 302, 303) lokalisiert sind und auf diese Druck ausüben können.

5. Pelotte (200, 400, 600, 700) nach einem der vorhergehenden Ansprüche, **dadurch gekennzeichnet, dass** das Grundelement (150, 850) des Pelottendruckelements (100, 800) mindestens ein Loch (120, 820) aufweist und wobei mindestens zwei Erhebungen (121, 122, 123, 124, 821, 822, 823, 824) auf dem Grundelement (150) am Rand (126) des mindestens einen Lochs (120, 820) positioniert sind und wobei optional die mindestens zwei Erhebungen (121, 122, 123, 124, 821, 822, 823, 824) über den das Loch (120, 820) überspannenden Steg (127, 128) miteinander verbunden sind.

6. Pelotte (200, 400, 600, 700) nach Anspruch 5, **dadurch gekennzeichnet, dass** am Rand (126) des Lochs (120, 820) mindestens zwei Paare von Erhebungen (121, 122, 123, 124, 821, 822, 823, 824) positioniert sind, die jeweils über einen das Loch (120, 820) überspannenden Steg (127, 128) miteinander verbunden sind, wobei sich die Stege (127, 128) der beiden Paare von Erhebungen (121, 122, 123, 124, 821, 822, 823, 824) kreuzen, bevorzugt etwa in der Mitte des Lochs (120, 820) kreuzen und wobei optional sich eine weitere Erhebung (125, 825) auf und/oder neben dem Kreuzungspunkt der beiden Stege (127, 128) befindet.

7. Pelotte (200, 400, 600, 700) nach Anspruch 5 oder 6, **dadurch gekennzeichnet, dass** die am Rand (126) des Lochs (120, 820) positionierten Erhebungen (121, 122, 123, 124, 821, 822, 823, 824) den mindestens zwei Reihen (101, 102, 103, 161, 162, 163, 171, 172,173, 181, 182, 183, 661, 662, 663, 671, 672, 673) von mindestens je drei Erhebungen (110, 610, 810) zugeordnet sind und wobei optional das Loch (120, 820) im Endbereich der mindestens zwei Reihen (101, 102, 103, 161, 162, 163, 171, 172,173, 181, 182, 183, 661, 662, 663, 671, 672, 673) von mindestensje drei Erhebungen (110, 610, 810) positioniert ist.

8. Pelotte (200, 400, 600, 700) nach einem der Ansprüche 5 bis 7, **dadurch gekennzeichnet, dass** das Loch (120, 820) im Pelottengrundkörper (201, 401, 402, 403, 501, 502, 503, 601, 602, 701) so positioniert ist, dass die am Rand (126) des Lochs (120, 820) positionierten Erhebungen (121, 122, 123, 124, 125, 821, 822, 823, 824, 825) im angelegten Zustand der Pelotte (200, 400, 600, 700) im Bereich eines Sehnenknochenansatzes (310) lokalisiert sind und auf diesen Druck ausüben können.

9. Pelotte (400, 600, 700) nach einem der vorhergehenden Ansprüche, **dadurch gekennzeichnet, dass** das Pelottendruckelement (100, 800) mindestens zwei zusätzliche Reihen (171, 172, 173, 181, 182, 183, 671, 672, 673) von mindestens je drei Erhebungen (110, 610, 810) aufweist, wobei die mindestens zwei zusätzlichen Reihen (171, 172, 173, 181, 182, 183, 671, 672, 673) von Erhebungen (110, 610, 810) versetzt in einem Winkelbereich von 75° bis 105° und/oder versetzt in einem Winkelbereich von 165° bis 205° zu den ersten mindestens zwei Reihen (161, 162, 163, 661, 662, 663) von Erhebungen (110, 610, 810) verlaufen und/oder mindestens zwei zusätzliche Reihen (171, 172, 173, 181, 182, 183, 671, 672, 673) von mindestens je drei Erhebungen (110, 610, 810) aufweist, wobei die mindestens zwei zusätzlichen Reihen (171, 172, 173, 181, 182, 183, 671, 672, 673) von Erhebungen (110, 610, 810) um etwa 180° versetzt zu den ersten mindestens zwei Reihen (161, 162, 163, 661, 662, 663) von Erhebungen (110, 610, 810) verlaufen und/oder mindestens vier zusätzliche Reihen (171, 172, 173, 181, 182, 183) von mindestens je drei Erhebungen (110, 610, 810) aufweist, wobei die mindestens vier zusätzlichen Reihen (171, 172, 173, 181, 182, 183) von Erhebungen (110, 610, 810) versetzt in einem Winkelbereich von 75° bis 105° und/oder versetzt in einem Winkelbereich von 165° bis 205° zu den ersten mindestens zwei Reihen (161, 162, 163) von Erhebungen (110, 610, 810) verlaufen.

10. Pelotte (700) nach einem der vorhergehenden Ansprüche, **dadurch gekennzeichnet, dass** das Pelottendruckelement (800) mindestens acht Reihen, bevorzugt zwölf Reihen, von mindestens je drei Erhebungen (810) aufweist, wobei die mindestens acht Reihen von Erhebungen (810) strahlenförmig aufeinander zulaufen und wobei optional sich in der Mitte der strahlenförmig aufeinander zulaufenden Reihen von Erhebungen (810) ein Loch (820) in dem Pelottendruckelement (800) befindet, wobei mindestens zwei Erhebungen (821, 822, 823, 824) auf dem Grundelement (850) am Rand des Lochs (820) positioniert sind.

11. Pelotte (200, 400, 600, 700) nach einem der vorhergehenden Ansprüche, **dadurch gekennzeichnet, dass** das Grundelement (150, 850) des Pelottendruckelements (100, 800) stegförmig oder plattenförmig ausgebildet ist und/oder die Erhebungen (110, 610, 810) zapfenförmig oder noppenförmig sind.

12. Pelotte (200, 400, 600, 700) nach einem der vorhergehenden Ansprüche, **dadurch gekennzeichnet, dass** der Pelottengrundkörper (201, 401, 402, 403, 501, 502, 503, 601, 602, 701) eine erste, dem Benutzer zugewandte Grundfläche (204) und eine zweite, dem Benutzer abgewandte Grundfläche (205) aufweist und wobei die Erhebungen (110, 610, 810) von dem im Bereich der zweiten, dem Benutzer abgewandten Grundfläche (205) positionierten Grundelements (150) in Richtung der ersten, dem Benutzer zugewandten Grundfläche (204) ragen.

13. Pelotte (200, 400, 600, 700) nach einem der vorhergehenden Ansprüche, **dadurch gekennzeichnet, dass** die Pelotte eine Ellenbogenpelotte, eine Schulterpelotte, eine Kniepelotte, eine Sprunggelenkspelotte, eine Rückenpelotte, eine Pelotte für den Armbereich oder eine Pelotte für den Bauchbereich ist.

14. Pelotte (200, 600) nach einem der vorhergehenden Ansprüche, **dadurch gekennzeichnet, dass** die Pelotte eine Ellenbogenpelotte ist, wobei der Grundkörper der Pelotte ein Loch zum Positionieren der Pelotte aufweist.

15. Orthese, Bandage (1000) oder Kompressionsbekleidung umfassend eine Pelotte (200, 400, 600, 700) nach einem der vorhergehenden Ansprüche.

## Claims

1. A pad (200, 400, 600, 700) for orthopedic aids or compression garments, comprising a pad base body (201, 401, 402, 403, 501, 502, 503, 601, 602, 701) of a first material and at least one pad pressure element (100, 800) of a second material, wherein the first material of the pad base body (201, 401, 402, 403, 501, 502, 503, 601, 602, 701) is softer than the second material of the pad pressure element (100, 800), wherein the pad pressure element (100, 800) has at least two rows (101, 102, 103, 161, 162, 163, 171, 172,173, 181, 182, 183, 661, 662, 663, 671, 672, 673) of at least three elevations (110, 610, 810) each, wherein the at least two rows (101, 102, 103, 161, 162, 163, 171, 172,173, 181, 182, 183, 661, 662, 663, 671, 672, 673) of elevations (110, 610, 810) do not extend in parallel at least in one portion, and wherein the elevations (110, 610, 810) protrude from a base element (150, 850) of the pad pressure element **characterized in that,** the pad pressure element (100, 800) is embedded in the pad base body (201, 401, 402, 403, 501, 502, 503, 601, 602, 701) at least in part and that two adjacent rows (101, 102, 103, 161, 162, 163, 171, 172,173, 181, 182, 183, 661, 662, 663, 671, 672, 673) of the at least two rows (101, 102, 103, 161, 162, 163, 171, 172,173, 181, 182, 183, 661, 662, 663, 671, 672, 673) of at least three elevations (110, 610, 810) each in the non-parallel portion extend at an angle of at least 3° and at most 25° to one another.

2. The pad (200, 400, 600, 700) according to claim 1, **characterized in that** the pad pressure element (100, 800) has three to six rows (101, 102, 103, 161, 162, 163, 171, 172,173, 181, 182, 183, 661, 662, 663, 671, 672, 673) of at least three elevations (110, 610, 810) each.

3. The pad (200, 400, 600, 700) according to any of the preceding claims, **characterized in that** the rows (101, 102, 103, 161, 162, 163, 171, 172,173, 181, 182, 183, 661, 662, 663, 671, 672, 673) each have four to twelve elevations (110, 610, 810).

4. The pad (200, 400, 600, 700) according to any of the preceding claims, **characterized in that** the at least two rows (101, 102, 103, 161, 162, 163, 171, 172,173, 181, 182, 183, 661, 662, 663, 671, 672, 673) of at least three elevations (110, 610, 810) each in the pad base body (201, 401, 402, 403, 501, 502, 503, 601, 602, 701) are positioned in such a way that they are located in the region of muscle strands (301, 302, 303) when the pad (200, 400, 600, 700) is applied and can exert pressure on said muscle strands.

5. The pad (200, 400, 600, 700) according to any of the preceding claims, **characterized in that** the base element (150, 850) of the pad pressure element (100, 800) has at least one hole (120, 820), and wherein at least two elevations (121, 122, 123, 124, 821, 822, 823, 824) are positioned on the base element (150) at the edge (126) of the at least one hole (120, 820) and wherein optionally the at least two elevations (121, 122, 123, 124, 821, 822, 823, 824) are connected to one another via the web (127, 128) spanning the hole (120, 820).

6. The pad (200, 400, 600, 700) according to claim 5, **characterized in that** at least two pairs of elevations (121, 122, 123, 124, 821, 822, 823, 824) are positioned at the edge (126) of the hole (120, 820) and are each connected to one another via a web (127, 128) spanning the hole (120, 820), wherein the webs (127, 128) of the two pairs of elevations (121, 122, 123, 124, 821, 822, 823, 824) intersect, preferably approximately in the center of the hole (120, 820) and wherein optionally another elevation (125, 825) is located on and/or next to the point of intersection of the two webs (127, 128).

7. The pad (200, 400, 600, 700) according claim 5 or 6, **characterized in that** the elevations (121, 122, 123, 124, 821, 822, 823, 824) positioned at the edge (126) of the hole (120, 820) are assigned to the at least two rows (101, 102, 103, 161, 162, 163, 171, 172,173, 181, 182, 183, 661, 662, 663, 671, 672, 673) of at least three elevations (110, 610, 810) each and wherein optionally the hole (120, 820) is positioned in the end region of the at least two rows (101, 102, 103, 161, 162, 163, 171, 172,173, 181, 182, 183, 661, 662, 663, 671, 672, 673) of at least three elevations (110, 610, 810) each.

8. The pad (200, 400, 600, 700) according to any of claims 5 to 7, **characterized in that** the hole (120, 820) in the pad base body (201, 401, 402, 403, 501, 502, 503, 601, 602, 701) is positioned in such a way that the elevations (121, 122, 123, 124, 125, 821, 822, 823, 824, 825) positioned at the edge (126) of the hole (120, 820) are located in the region of a tendon-bone insertion (310) when the pad (200, 400, 600, 700) is applied and can exert pressure on said tendon-bone insertion.

9. The pad (400, 600, 700) according to any of the preceding claims, **characterized in that** the pad pressure element (100, 800) has at least two additional rows (171, 172, 173, 181, 182, 183, 671, 672, 673) of at least three elevations (110, 610, 810) each, wherein the at least two additional rows (171, 172, 173, 181, 182, 183, 671, 672, 673) of elevations (110, 610, 810) extend so as to be offset in an angular range of from 75° to 105° and/or so as to be offset in an angular range of from 165° to 205° with respect to the first at least two rows (161, 162, 163, 661, 662, 663) of elevations (110, 610, 810) and/or has at least two additional rows (171, 172, 173, 181, 182, 183, 671, 672, 673) of at least three elevations (110, 610, 810) each, wherein the at least two additional rows (171, 172, 173, 181, 182, 183, 671, 672, 673) of elevations (110, 610, 810) extend so as to be offset by approximately 180° with respect to the first at least two rows (161, 162, 163, 661, 662, 663) of elevations (110, 610, 810) and/or has at least four additional rows (171, 172, 173, 181, 182, 183) of at least three elevations (110, 610, 810) each, wherein the at least four additional rows (171, 172, 173, 181, 182, 183) of elevations (110, 610, 810) extend so as to be offset in an angular range of from 75° to 105° and/or so as to be offset in an angular range of from 165° to 205° with respect to the first at least two rows (161, 162, 163) of elevations (110, 610, 810).

10. The pad (700) according to any of the preceding claims, **characterized in that** the pad pressure element (800) has at least eight rows, preferably twelve rows, of at least three elevations (810) each, wherein the at least eight rows of elevations (810) converge in the manner of rays and wherein optionally a hole (820) is located in the pad pressure element (800) in the center of the rows of elevations (810) that converge in the manner of rays, wherein at least two elevations (821, 822, 823, 824) are positioned on the base element (850) at the edge of the hole (820).

11. The pad (200, 400, 600, 700) according to any of the preceding claims, **characterized in that** the base element (150, 850) of the pad pressure element (100, 800) is web-shaped or plate-shaped and/or the elevations (110, 610, 810) are stud-shaped or knob-shaped.

12. The pad (200, 400, 600, 700) according to any of the preceding claims, **characterized in that** the pad base body (201, 401, 402, 403, 501, 502, 503, 601, 602, 701) has a first base surface (204) facing the user and a second base surface (205) facing away from the user, and wherein the elevations (110, 610, 810) project from the base element (150) positioned in the region of the second base surface (205), which faces away from the user, in the direction of the first base surface (204), which faces the user.

13. The pad (200, 400, 600, 700) according to any of the preceding claims, **characterized in that** the pad is an elbow pad, a shoulder pad, a knee pad, an ankle pad, a back pad, a pad for the arm region, or a pad for the abdominal region.

14. The pad (200, 600) according to any of the preceding claims, **characterized in that** the pad is an elbow pad, wherein the base body of the pad comprises a hole for positioning the pad.

15. An orthosis, bandage (1000), or compression garment comprising a pad (200, 400, 600, 700) according to any of the preceding claims.

## Revendications

1. Un coussinet (200, 400, 600, 700) pour aides orthopédiques ou vêtements de compression, comprenant un corps de base du coussinet (201, 401, 402, 403, 501, 502, 503, 601, 602, 701) d'un premier matériau et au moins un élément de pression du coussinet (100, 800) d'un second matériau, dans lequel le premier matériau du corps de base du coussinet (201, 401, 402, 403, 501, 502, 503, 601, 602, 701) est plus souple que le second matériau de l'élément de pression du coussinet (100, 800), dans lequel l'élément de pression du coussinet (100, 800) a au moins deux rangées (101, 102, 103, 161, 162, 163, 171, 172,173, 181, 182, 183, 661, 662, 663, 671, 672, 673) d'au moins trois élévations (110, 610, 810) chacune, dans laquelle les au moins deux rangées (101, 102, 103, 161, 162, 163, 171, 172,173, 181, 182, 183, 661, 662, 663, 671, 672, 673) des élévations (110, 610, 810) ne s'étendent pas parallèlement au moins dans une partie, et dans lequel les élévations (110, 610, 810) dépassent d'un élément de base (150, 850) de l'élément de pression du coussinet **caractérisé en ce que**, l'élément de pression du coussinet (100, 800) est intégré dans le corps de base du coussinet (201, 401, 402, 403, 501, 502, 503, 601, 602, 701) au moins dans une partie et que deux rangées adjacentes (101, 102, 103, 161, 162, 163, 171, 172,173, 181, 182, 183, 661, 662, 663, 671, 672, 673) des au moins deux rangées (101, 102, 103, 161, 162, 163, 171, 172,173, 181, 182, 183, 661, 662, 663, 671, 672, 673) d'au moins trois élévations (110, 610, 810) chacune dans la partie non parallèle s'étendent selon un angle d'au moins 3° et d'au plus 25° l'une par rapport à l'autre.

2. Le coussinet (200, 400, 600, 700) selon la revendication 1, **caractérisé en ce que** l'élément de pression du coussinet (100, 800) a trois à six rangées (101, 102, 103, 161, 162, 163, 171, 172,173, 181, 182, 183, 661, 662, 663, 671, 672, 673) d'au moins trois élévations (110, 610, 810) chacune.

3. Le coussinet (200, 400, 600, 700) selon l'une quelconque des revendications précédentes, **caractérisé en ce que** les rangées (101, 102, 103, 161, 162, 163, 171, 172,173, 181, 182, 183, 661, 662, 663, 671, 672, 673) ont chacune de quatre à douze élévations (110, 610, 810).

4. Le coussinet (200, 400, 600, 700) selon l'une quelconque des revendications précédentes, **caractérisé en ce que** les au moins deux rangées (101, 102, 103, 161, 162, 163, 171, 172,173, 181, 182, 183, 661, 662, 663, 671, 672, 673) d'au moins trois élévations (110, 610, 810) chacune dans le corps de base du coussinet (201, 401, 402, 403, 501, 502, 503, 601, 602, 701) sont positionnées au moyen de sorte qu'elles sont située dans la région des brins musculaires (301, 302, 303) lorsque le coussinet (200, 400, 600, 700) est appliqué et qu'elles peuvent exercer une pression sur lesdits brins musculaires.

5. Le coussinet (200, 400, 600, 700) selon l'une quelconque des revendications précédentes, **caractérisé en ce que** l'élément de base (150, 850) de l'élément de pression du coussinet (100, 800) a au moins un trou (120, 820), et dans lequel au moins deux élévations (121, 122, 123, 124, 821, 822, 823, 824) sont positionnées sur l'élément de base (150) au bord (126) du au moins trou (120, 820) et dans lequel optionnellement les au moins deux élévations (121, 122, 123, 124, 821, 822, 823, 824) sont connectées l'une à l'autre via la barre (127, 128) enjambant le trou (120, 820).

6. Le coussinet (200, 400, 600, 700) selon la revendication 5, **caractérisé en ce qu'**au moins deux paires d'élévations (121, 122, 123, 124, 821, 822, 823, 824) sont positionnées au bord (126) du trou (120, 820) et sont chacune connectées l'une à l'autre via une barre (127, 128) enjambant le trou (120, 820), dans lequel les barres (127, 128) des deux paires d'élévations (121, 122, 123, 124, 821, 822, 823, 824) se croisent, préférablement approximativement au centre du trou (120, 820) et dans lequel optionnellement une autre élévation (125, 825) est située sur et/ou à côté du point d'intersection des deux barres (127, 128).

7. Le coussinet (200, 400, 600, 700) selon la revendication 5 ou 6, **caractérisé en ce que** les élévations (121, 122, 123, 124, 821, 822, 823, 824) positionnées au bord (126) du trou (120, 820) sont affectées aux au moins deux rangées (101, 102, 103, 161, 162, 163, 171, 172,173, 181, 182, 183, 661, 662, 663, 671, 672, 673) d'au moins trois élévations (110, 610, 810) chacune et dans lequel optionnellement le trou (120, 820) est positionné dans la région d'extrémité des au moins deux rangées (101, 102, 103, 161, 162, 163, 171, 172,173, 181, 182, 183, 661, 662, 663, 671, 672, 673) d'au moins trois élévations (110, 610, 810) chacune.

8. Le coussinet (200, 400, 600, 700) selon l'une quelconque des revendications 5 à 7, **caractérisé en ce que** le trou (120, 820) dans le corps de base du coussinet (201, 401, 402, 403, 501, 502, 503, 601, 602, 701) est positionné au moyen de sorte que les élévations (121, 122, 123, 124, 125, 821, 822, 823, 824, 825) positionnées au bord (126) du trou (120, 820) sont situées dans la région d'une insertion tendon-os (310) lorsque le coussinet (200, 400, 600, 700) est appliqué et peuvent exercer une pression sur ladite insertion tendon-os.

9. Le coussinet (400, 600, 700) selon l'une quelconque des revendications précédentes, **caractérisé en ce que** l'élément de pression du coussinet (100, 800) a au moins deux rangées supplémentaires (171, 172, 173, 181, 182, 183, 671, 672, 673) d'au moins trois élévations (110, 610, 810) chacune, dans lesquelles les au moins deux rangées supplémentaires (171, 172, 173, 181, 182, 183, 671, 672, 673) d'élévations (110, 610, 810) s'étendent de manière à être décalées dans une plage angulaire de 75° à 105° et/ou de manière à être décalées dans une plage angulaire de 165° à 205° par rapport aux au moins deux premières rangées (161, 162, 163, 661, 662, 663) d'élévations (110, 610, 810) et/ou a au moins deux rangées supplémentaires (171, 172, 173, 181, 182, 183, 671, 672, 673) d'au moins trois élévations (110, 610, 810) chacune, dans laquelle les au moins deux rangées supplémentaires (171, 172, 173, 181, 182, 183, 671, 672, 673) d'élévations (110, 610, 810) s'étendent de manière à être décalées d'environ 180° par rapport aux premières au moins deux rangées (161, 162, 163, 661, 662, 663) d'élévations (110, 610, 810) et/ou a au moins quatre rangées supplémentaires (171, 172, 173, 181, 182, 183) d'au moins trois élévations (110, 610, 810) chacune, dans lesquelles les au moins quatre rangées supplémentaires (171, 172, 173, 181, 182, 183) d'élévations (110, 610, 810) s'étendent de manière à être décalées dans une plage angulaire de 75° à 105° et/ou de manière à être décalées dans une plage angulaire de 165° à 205° par rapport aux premières au moins deux rangées (161, 162, 163) d'élévations (110, 610, 810).

10. Le coussinet (700) selon l'une quelconque des revendications précédentes, **caractérisé en ce que** l'élément de pression du coussinet (800) a au moins huit rangées, préférablement douze rangées, d'au moins trois élévations (810) chacune, dans lequel les au moins huit rangées d'élévations (810) convergent à la manière de rayons et dans lequel, optionnellement, un trou (820) est situé dans l'élément de pression du coussinet (800) au centre des rangées d'élévations (810) qui convergent à la manière de rayons, dans lequel au moins deux élévations (821, 822, 823, 824) sont positionnées sur l'élément de base (850) au bord du trou (820).

11. Le coussinet (200, 400, 600, 700) selon l'une quelconque des revendications précédentes, **caractérisé en ce que** l'élément de base (150, 850) de l'élément de pression du coussinet (100, 800) est en forme de barre ou de plaque et/ou les élévations (110, 610, 810) sont en forme de goujon ou de bouton.

12. Le coussinet (200, 400, 600, 700) selon l'une quelconque des revendications précédentes, **caractérisé en ce que** le corps de base du coussinet (201, 401, 402, 403, 501, 502, 503, 601, 602, 701) a une première surface de base (204) orientée vers l'utilisateur et une deuxième surface de base (205) orientée à l'opposé de l'utilisateur, et dans lequel les élévations (110, 610, 810) font saillie à partir de l'élément de base (150) positionné dans la région de la seconde surface de base (205), qui est orientée à l'opposé de l'utilisateur, en direction de la première surface de base (204), qui est orientée vers l'utilisateur.

13. Le coussinet (200, 400, 600, 700) selon l'une quelconque des revendications précédentes, **caractérisé en ce que** le coussinet est un coussinet de coude, un coussinet d'épaule, un coussinet de genou, un coussinet de cheville, un coussinet de dos, un coussinet pour la région du bras, ou un coussinet pour la région abdominale.

14. Le coussinet (200, 600) selon l'une quelconque des revendications précédentes, **caractérisé en ce que** le coussinet est un coussinet de coude, dans lequel le corps de base du coussinet comprend un trou pour le positionnement du coussinet.

15. Une orthèse, un bandage (1000), ou un vêtement de compression comprenant un coussinet (200, 400, 600, 700) selon l'une quelconque des revendications précédentes.
